# EUROPÄISCHE PATENTSCHRIFT

(11) **EP 2 888 971 B1**
(45) Veröffentlichungstag und Bekanntmachung des Hinweises auf die Patenterteilung: **04.09.2019**
(21) Anmeldenummer: 14197205.9
(22) Anmeldetag: 10.12.2014
(51) Int. Cl.: A45D 19/02, A45D 24/28, A46B 11/00

(54) **Applikator mit borstenbesetzten Kartuschen**
Applicator with cartridges equipped with brushes
Applicateur doté de cartouches garnies de soie

(30) Priorität: 27.12.2013 DE 202013011647 U
(43) Veröffentlichungstag der Anmeldung: 01.07.2015
(73) Patentinhaber: GEKA GmbH, 91572 Bechhofen (DE)
(72) Erfinder: Hartstock-Martin, Karl, 91522 Ansbach (DE)
(74) Vertreter: Misselhorn, Hein-Martin

(56) Entgegenhaltungen:
- WO-A1-98/51183
- CN-A- 101 637 332
- DE-A1- 2 830 480
- FR-A1- 2 772 569
- FR-A3- 2 588 734
- US-A- 1 742 157
- US-A- 4 139 312
- US-A1- 2004 035 435

## Beschreibung

Die Erfindung besteht aus einem Kosmetik- oder Pharmazieapplikator nach dem Oberbegriff des Anspruchs 1.

Dünnflüssige pharmazeutische oder kosmetische Zusammensetzungen werden häufig mit Hilfe eines Pinsels aufgebracht.

Schwierigkeiten bereitet dies nicht zuletzt dort, wo pharmazeutische oder kosmetische Zusammensetzungen auf die behaarte Haut aufzubringen sind. Ein typisches Beispiel hierfür sind Haarwuchsmittel.

Das Aufbringen solcher Substanzen auf die behaarte Haut führt im Regelfall dazu, dass deutlich mehr Substanz zum Einsatz kommen muss, als am Ende der Haut zu Gute kommt, denn ein guter Teil der aufzutragenden Substanz bleibt in den Haaren hängen. Die kosmetische Substanz ist damit verloren bzw. verklebt die Haare sogar und wirkt sich dadurch störend aus.

Um pharmazeutische oder kosmetische Substanzen auf die behaarte Haut aufzutragen, wird alternativ zu einem Pinsel häufig ein Wattestäbchen verwendet. Es wird mit der aufzutragenden Substanz beträufelt und dann wird versucht, möglichst unter Umgehung der Haare, auf die behaarte Haut einzuwirken. Eine solche Vorgehensweise verringert immerhin die Menge des Pharmazeutikums oder Kosmetikums, das in den Haaren hängen bleibt. Dennoch ist der Verbrauch nach wie vor relativ hoch, da das Wattestäbchen relativ stark saugt und daher einen guten Teil der pharmazeutischen oder kosmetischen Substanz aufnimmt, aber nicht wieder abgibt.

Aufgrund dessen ist auch schon daran gedacht worden, pharmazeutische oder kosmetische Substanzen auf die behaarte Haut mit Hilfe eines relativ harten Pinsels aufzutragen. Hiermit lässt sich die Menge des Kosmetikums oder Pharmazeutikums, das in den Haaren hängen bleibt und nicht der Haut zu Gute kommt, reduzieren. Solange man von dem Pinsel nur die Pinselspitze mit dem aufzutragenden Kosmetikum oder Pharmazeutikum benetzt, lässt sich auch über die Wattestäbchen der Substanzverbrauch verringern. Ein Problem stellt aber die Tatsache dar, dass die zum Auftragen verwendeten Pinsel im Regelfall nicht verworfen, sondern gereinigt werden und dann beispielsweise mühsam getrocknet werden müssen. Und eine solche Reinigung ist auch hygienisch nicht ganz unbedenklich, denn ein Pinsel lässt sich fast nie hundertprozentig reinigen.

Zudem gilt es insbesondere bei teuren pharmazeutischen oder kosmetischen Substanzen, wie beispielsweise Haarwuchsmitteln, den Verbrauch weiter zu reduzieren.

WO 98/51183 A1 offenbart einen Kosmetik- oder Parmazieapplikator nach dem Oberbegriff des Anspruchs 1.

Die Aufgabe wird durch einen Kosmetik- oder Pharmazieapplikator mit dem Merkmal des Anspruchs 1 gelöst.

Der Vorrat des aufzutragenden kosmetischen oder pharmazeutischen Fluids wird bei dem erfindungsgemäßen Applikatoraußerhalb des Borstenbesatzes bereitgehalten. Der erfindungsgemäße Applikator besitzt einen Pumpmechanismus zur Ausgabe des Fluids in den Borstenbesatz, vorzugsweise durch eine oder mehrere Borsten hindurch.

Dabei ist der Borstenbesatz des Applikators fester Bestandteil einer den Vorrat des Fluids bereithaltenden Kartusche, die zusammen mit dem Borstenbesatz einen von den sonstigen Komponenten des Applikators unabhängigen, separat handhabbaren Bestandteil bildet. Diese Kartusche kann vorzugsweise werkzeuglos in eine hierfür vorgesehene Kammer des Applikators ein- und wieder ausgebaut werden. Mit Hilfe des Pumpmechanismus kann ggf. eine sehr feinfühlige Dosierung vorgenommen werden. Die besagte Dosierung kann entweder dadurch erreicht werden, dass die Kartusche jeweils nur so viel Fluid enthält, wie für eine Anwendung erforderlich ist. Alternativ kann der Pumpmechanismus so angetrieben werden, dass er, einmal ausgelöst, nur eine vorher bestimmte Menge des Fluids aus der Kartusche ausgibt, die eine Fluidmenge bevorratet, welche für mehrere Anwendungen ausreicht.

Das Fluid wird in den Borstenbesatz ausgegeben. Hierdurch wird verhindert, dass Fluid "daneben" geht, noch bevor der Pinsel die Hautpartie erreicht hat, auf die appliziert werden soll. Denn der erfindungsgemäße Applikator macht es, jedenfalls in seiner motorbetriebenen Variante, möglich, erst den Pinsel auf die zu behandelnde Hautpartie aufzusetzen und dann den Pumpmechanismus in Gang zu setzen. Das verbessert den Auftrag sowohl beim Applizieren von Substanz im Bereich der behaarten Haut als auch im unbehaarten Bereich spürbar.

Noch besser zur Geltung kommt die Erfindung, wenn das Fluid nicht im Bereich des Borstenfußes in den Borstenbesatz ausgegeben wird, sondern wenn das Fluid über zumindest eine innen hohle Borste in den Bereich der Borstenspitzen ausgegeben wird. Dann wird das Fluid so dicht, wie es überhaupt möglich ist, an die zu behandelnde Hautpartie herangeführt, so dass nur noch minimale Verluste zu befürchten sind. Insbesondere tritt bei einer solchen Ausgestaltung auch kein Verlust dadurch auf, dass ein nennenswerter Anteil des Fluids im Borstenbesatz des Pinsels hängenbleibt. Das Fluid wird über die hohle Borste direkt an der oder den Borstenspitzen ausgegeben, haftet also sofort und auf kürzestem Wege an der Kopfhaut an. Die Borsten dienen dadurch nicht länger zum Zuführen des Fluids in den Bereich der Kopfhaut, also als ein Mittel, das das Fluid zunächst zwischen den Borsten speichert und dann, bei Berührung mit der behaarten Haut, mehr oder minder vollständig wieder abgibt. Stattdessen werden die Borsten im Wesentlichen nur noch zum Einmassieren und Verteilen des bereits an der Kopfhaut anhaftenden Fluids verwendet.

Mit Hilfe des erfindungsgemäßen Applikators wird auch das Problem der dauerhaften Abdichtung des Raums, in dem das Fluid gespeichert wird, gut gelöst. Denn bei sämtlichen Arten von Applikatoren, die einen Vorrat des zu applizierenden Fluids beherbergen, tritt immer wieder das Problem auf, dass sich im laufenden Betrieb nach geraumer Zeit Undichtigkeiten ergeben, weil die betreffenden Dichtungen, die natürlich nicht extrem hochpreisig sein können, um die Kosten des Applikators nicht in einen unakzeptablen Bereich zu treiben, verschleißen. Mit der erfindungsgemäßen Applikatorlösung wird dem entgegengewirkt. Selbst wenn ein Dichtungsverschleiß eintritt, muss nicht der gesamte Applikator verworfen werden, sondern es reicht, die Kartusche zu erneuern.

Aus diesem Grund, aber auch aus Hygienegründen, ist es besonders empfehlenswert, wenn die Kartusche eine Einwegkartusche ist. Dann lassen sich die Vorteile der Erfindung besonders gut ausspielen. Dadurch, dass der Borstenbesatz fester Bestandteil der Kartusche ist, wird jeweils nicht nur die Kartusche ausgewechselt, sondern automatisch auch der Borstenbesatz. Auf diese Art und Weise ist der Applikator nicht nur stets "gut gefüllt", sondern bietet auch stets einen hygienischen und gut in Form befindlichen Borstenbesatz. Die Borsten können weicher ausgelegt werden als bei einem Applikator, der dutzende von Applikationen überstehen muss, ohne dass sich die Borsten verformen dürfen. Die an und für sich mit einem recht weichen Borstenbesatz verbundenen Nachteile lassen sich hier gut beherrschen, denn über den endlichen Kartuscheninhalt lässt sich sehr genau vorherbestimmen, wie viele Applikationen die Borsten ertragen müssen, ohne übermäßigen Deformationen zu unterliegen.

Besonders vorteilhaft ist die Verwendung von Einwegkartuschen auch dort, wo besonders hohe Hygiene-Voraussetzungen gestellt werden oder sogar ein steriler Einsatz erforderlich ist. In diesem Fall kommen Einwegkartuschen zur Verwendung, deren Speichervolumen genau oder jedenfalls im Wesentlichen so groß ist, dass nur das für eine einzige Anwendung benötigte Fluid bevorratet ist. Nach der einzigen Anwendung wird die Kartusche mitsamt dem Borstenbesatz sofort verworfen und für die nächste Anwendung durch eine neue Kartusche mit einem ebenfalls neuen Borstenbesatz ersetzt.

Auf diese Art und Weise kann Sterilität sichergestellt werden. Es ist sogar noch einfacher, eine fast hundertprozentige Sterilität zu gewährleisten, als bei einem separaten Pinsel, den man in einen Vorrat eintaucht. Denn hier bei dem erfindungsgemäßen System bleibt der Borstenbesatz bis zu seinem Auftreffen auf die Kopfhaut steril, erst dann wird das Fluid ausgegeben. Die Notwendigkeit eines Eintauchens in ein Fluid und die damit verbundene Gefahr, dass eventuell auch mehrfach eingetaucht wird und dadurch Keime verschleppt werden, entfällt.

Die Kartusche beherbergt jeweils in ungebrauchtem Zustand eine Blase, die aus einer hermetisch dicht verschlossenen Hülle besteht, die mit dem zu applizierenden Fluid befüllt ist. Auf diese Art und Weise wird ebenfalls erhöhte Sterilität sichergestellt und/oder negative Umgebungseinwirkungen auf das Fluid (insbesondere Luftzutritt) werden so gut wie möglich ausgeschlossen.

Zweckmäßigerweise weist die Kartusche mindestens einen in ihren Innenraum, der zur Aufnahme der Blase bestimmt ist, hineinragenden Dorn auf. Dieser Dorn sticht die Blase auf, sobald der Pumpmechanismus zur Ausgabe des Kosmetikums in Gang gesetzt wird und dann die Blase gegen den Dorn presst. Besonders bevorzugt ist es, wenn der Dorn einen nicht-runden, vorzugsweise kreuzförmigen Querschnitt aufweist. Ein solcher Querschnitt bringt es mit sich, dass die Blase nicht etwa gegen den Außenumfang des Dorns abdichtet, so dass nur über den hohlen Innenquerschnitt des Dorns Fluid abgegeben werden kann, sondern dass Fluid aus der Blase in den gesamten Innenraum der Kartusche austreten kann. Eine solche Ausgestaltung ist immer dann sinnvoll, wenn das Fluid aus der Blase nicht nur über eine einzige hohle Borste nach außen ausgegeben werden soll, sondern über eine Mehrzahl von innen hohlen Borsten, die jeweils eine Verbindung zwischen dem Innenraum der Kartusche 3 und der Umgebung herstellen.

Die Kartusche besteht im Wesentlichen aus einem härteren ersten Kunststoff, der zumindest die Umfangswände und eine Stirnwand der Kartusche ausbildet, und einem weicheren, vorzugsweise weich- oder gummielastischen zweiten Kunststoff, der die Borsten bildet, die unlösbar mit der Kartusche verbunden sind. Die Kartusche und die daran angespritzten Borsten werden also in zwei aufeinanderfolgenden Spritzgussschritten hergestellt. Eine solche Produktionsart und die daraus folgende Produktbeschaffenheit erlauben es, sowohl die Kartusche als auch die Borsten an ihre jeweilige Funktion optimal anzupassen. Die Kartusche muss aus härterem Kunststoff sein, um formbeständig zu sein und auch einem gewissen Innendruck standzuhalten. Die Borsten hingegen müssen oft möglichst sehr weich und elastisch sein, um bei Auftrag auf empfindliche behaarte Hautpartien nicht unnötig zu reizen oder ein unangenehmes Gefühl zu verursachen.

Vorzugsweise umfasst der Borstenbesatz zumindest eine und idealerweise nur eine Borste, die innen hohl ist und über den so gebildeten Borstenkanal den das zu applizierende Fluid beherbergenden Innenraum der Kartusche mit der Umgebung des Kosmetikpinsels verbindet. Auf diese Art und Weise kann besonders effizient Fluid ausgegeben werden. Das Fluid tritt an der äußersten Spitze der mindestens einen bzw. der nur einen Borste aus. Der Borstenbesatz hat also keine Gelegenheit, mit dem Fluid getränkt zu werden und einen guten Teil des Fluids zurückzuhalten, anstatt an die Haut abzugeben. Stattdessen dient der Borstenbesatz überwiegend zum Verteilen des Fluids auf der Haut, wobei meist nur die Spitzen der den Borstenbesatz bildenden Borsten (ca. das äußerste Drittel der Borsten) wirklich mehr als nur unwesentlich mit dem aufzutragenden Fluid in Kontakt kommen. Diese Art der Ausgestaltung ist besonders dann vorteilhaft, wenn der Kartuschenkörper und sein stirnseitiger Borstenbesatz einstückig und einstofflich aus dem gleichen, einheitlichen Kunststoff gespritzt werden, vorzugsweise in einem Schuss. Auf diese Art und Weise lässt sich nämlich am einfachsten mit Hilfe eines dünnen Dorns (Durchmesser ≤ 1,5 mm, besser ≤ 1 mm) eine innen hohle Borste spritzen.

Vorzugsweise wird der Borstenkanal in Abstimmung auf die Viskosität und die Kapillarwirkung des Fluids so eng ausgebildet, dass das in der Kartusche enthaltene Fluid durch die Kapillarwirkung des Borstenkanals daran gehindert wird, über den Borstenkanal unbeabsichtigt nach außen auszutreten, solange der Kartuscheninhalt nicht unter Überdruck gesetzt wird. Auf diese Art und Weise kann ein unbeabsichtigtes Auslaufen des Applikators vermieden werden, der z. B. während einer Behandlungspause nicht im Kopfstand abgestellt, sondern unachtsam auf seiner Seite abgelegt worden ist.

Für andere Einsatzfälle ist es besonders günstig, wenn die Innenseite der Stirnwand der Kartusche zumindest teilweise mit einer Schicht aus dem die Borsten bildenden Kunststoff bedeckt ist, wobei diese Schicht über örtliche Perforation des die Kartusche bildenden Kunststoffs mit den frei nach außen ragenden Borsten einstofflich und einstückig in Verbindung steht.

Auf diese Art und Weise kann der Borstenbesatz nicht nur sehr effektiv hergestellt werden, sondern er kann insbesondere auch sicher an der Kartusche verankert werden, gerade dann, wenn er aus einem anderen Material besteht als der Kartuschenkörper. Zweckmäßigerweise besitzt die Kartusche auf ihrer dem Borstenabsatz abgewandten Seite einen nach Art eines Kolbens verschiebbar geführten Boden bzw. Kartuschenkolben. Vorzugsweise ist dieser Kartuschenkolben aus einem dritten Kunststoff gefertigt, dessen Flexibilität idealerweise zwischen der Flexibilität des die Borsten bildenden Kunststoffs und der Flexibilität des die Umfangswand der Kartusche bildenden Kunststoffs liegt. Auf diese Art und Weise kann der Kartuschenkolben besonders gut federnd ausgebildet werden, so dass er nach seiner Montage in dem die Umfangswand der Kartusche bildenden Bereich elastisch vorgespannt und damit dicht gegen diesen anliegt.

Eine bevorzugte Ausführungsform sieht vor, dass die die Kartuschen aufnehmende Kammer des Pinsels zugänglich ist, indem ein Teil des Applikatorkörperoberteils um eine im Wesentlichen zur Applikatorlängsachse senkrechte Scharnierachse gegenüber dem Applikatorhauptkörper aufgeklappt werden kann. Durch ein solches Aufklappen lässt sich sehr schnell eine bequem zugängliche Öffnung zum Herausnehmen und Wiedereinsetzen von Kartuschen schaffen. Ein umständliches Aufschrauben entfällt.

Für viele Anwendungsfälle ist es besonders zweckmäßig, wenn im Applikator ein Vibrator eingebaut ist, der den Borstenbesatz bzw. den gesamten Applikator vibrieren lässt. Ideal ist ein Masseschwinger, der Vibrationen schräg oder im Wesentlichen senkrecht zur Applikatorlängsachse induziert, vorzugsweise mit einer Frequenz von mehr als 6 Hz und einer Amplitude von weniger als 1 mm.

Unabhängiger Schutz wird auch für ein System zum Auftrag eines Pharmazeutikums oder eines Kosmetikums beansprucht, bestehend aus einem Applikator nach einem der Ansprüche 1 - llsowie einer Mehrzahl von werkzeuglos nicht nachfüllbaren Kartuschen mit einem an diese angeformten Borstenbesatz, die jeweils einen Vorrat des aufzutragenden Pharmazeutikums oder Kosmetikums enthalten und an die Kartuschenaufnahmekammer des Applikators angepasst sind.

Idealerweise wird dieses System durch eine Blisterverpackung erweitert, die mehrere Kartuschen zu einem Kartuschenvorrat zusammengefasst hält. Dabei ist die Blisterverpackung so gestaltet, dass jede der Kartuschen einzeln aus der vorzugsweise einseitig klarsichtigen oder idealerweise rückseitig durch eine Metallfolie versiegelten Blisterverpackung entnommen werden kann, ohne die anderen Kartuschen bereits einem weitgehend ungehinderten Luftzutritt oder einer weitgehend ungehinderten Einwirkung von Umgebungseinflüssen auszusetzen.

Weitere Ausgestaltungsmöglichkeiten, Vorteile und Wirkungsweisen der Erfindung lassen sich der nachfolgenden Schilderung verschiedener Ausführungsbeispiele der Erfindung anhand der Figuren entnehmen.

Die Fig. 1 zeigt ein erstes Ausführungsbeispiel eines Applikators, welches nicht Teil der Erfindung ist, im Schnitt entlang der Längsachse L.

Die Fig. 1a zeigt einen Detailausschnitt aus der Fig. 1.

Die Fig. 2 zeigt eine einzelne Darstellung einer Kartusche für den erfindungsgemäßen Applikator entlang der Längsachse.

Die Fig. 3 zeigt eine einzelne Darstellung einer Kartusche für den erfindungsgemäßen Applikator, wie sie als weitere Alternative verwendet wird, entlang der Längsachse.

Die Fig. 4 zeigt ein Ausführungsbeispiel des erfindungsgemäßen Applikators von oben her gesehen.

Die Fig. 5 zeigt ein Ausführungsbeispiel des erfindungsgemäßen Applikators von der Seite her gesehen.

Die Fig. 6 zeigt ein Ausführungsbeispiel des erfindungsgemäßen Applikators von unten her gesehen.

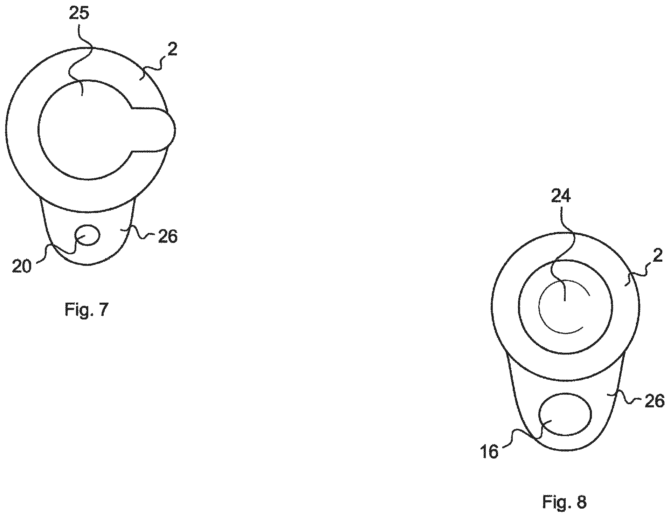

Die Fig. 7 zeigt ein Ausführungsbeispiel des erfindungsgemäßen Applikators frontal von vorne her gesehen.

Die Fig. 8 zeigt ein Ausführungsbeispiel des erfindungsgemäßen Applikators frontal von hinten her gesehen.

Die Fig. 9 zeigt eine Einzelansicht der Kartusche, wie sie im Rahmen des ersten Ausführungsbeispiels beschrieben worden ist.

Die Fig. 10 zeigt die Kartusche gemäß Fig. 9 bei aufgesetzter Kappe.

Die Fig. 11 zeigt ein anderes Ausführungsbeispiel eines erfindungsgemäßen Applikators schräg von der Seite her gesehen in betriebsfertigem Zustand.

Die Fig. 12 zeigt den Applikator gemäß Fig. 11 in aufgeklapptem Zustand beim Nachfüllen einer Kartusche.

Die Fig. 13 zeigt ein Blisterpack, der eine Anzahl von Kartuschen luftdicht geschützt bevorratet hält, wobei eine der Kartuschen bereits durch Herausdrücken entnommen worden ist.

Die Fig. 14 zeigt ein Beispiel für eine Kappe.

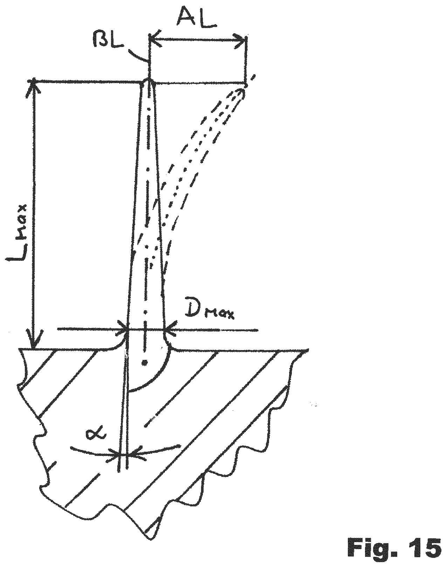

Die Fig. 15 erläutert mit Geltung für alle Ausführungsbeispiele, was vorzugsweise unter einer Borste im Sinne der Erfindung zu verstehen sein soll.

Die Fig. 16 macht einen Vorschlag zur besseren Abdichtung des Applikatorkörperoberteils ggü. dem Applikatorhauptkörper für das von Fig. 12 gezeigte Ausführungsbeispiel.

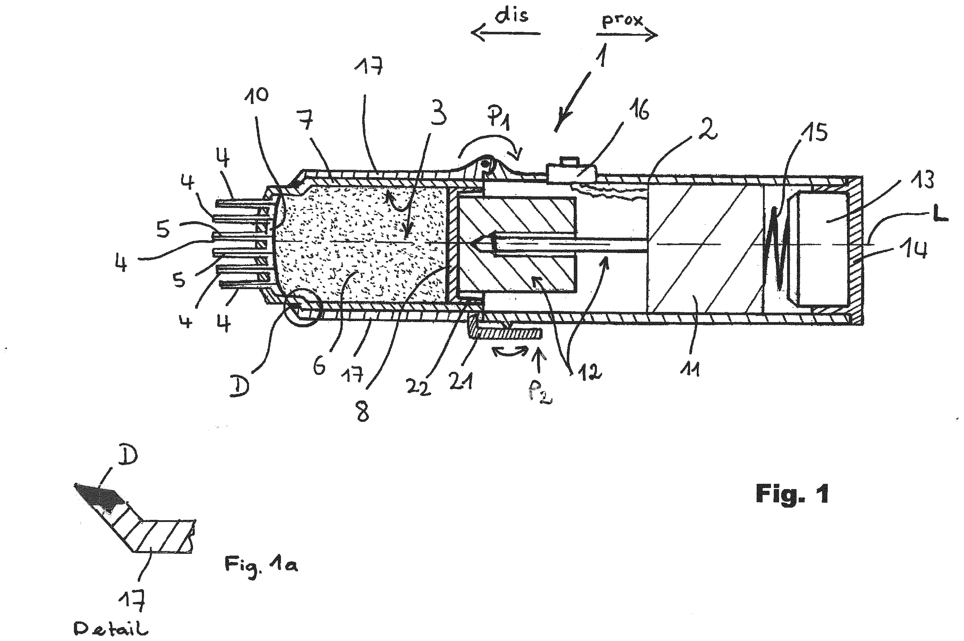

Den besten Überblick über das Prinzip eines Applikators des vorliegenden Kontextes gibt die Fig. 1.

Wie man sehen kann, besteht der Applikator 1 aus einem Applikatorhauptgehäuse 2 und einem Applikatorgehäuseoberteil 17.

Vorzugsweise ist es das Applikatorhauptgehäuse 2, das mittels eines normalerweise separat entfernbaren Batteriefachdeckels 14 eine Batterie 13 und einen Motor 11 beherbergt. Die Batterie 13 ist im Regelfall über u. a. ein Federelement 15 elektrisch mit dem Motor verbunden.

Betätigt wird der Motor 11 über den Schalter 16, bei dem es sich im Regelfall um einen Mikroschalter handelt, der ebenfalls im Applikatorhauptgehäuse 2 untergebracht ist, hier aber nicht im Detail gezeigt wird.

Motor 11 treibt ein Druckorgan 12 an, das hier aus einem als Druckstück zu bezeichnenden Körper mit einem Innengewinde besteht, in das eine von dem Motor angetriebene Gewindestange eingreift. Je nach Drehrichtung des Motors bewegt sich das besagte Druckstück vor oder zurück. Bewegt sich das Druckstück vor, in Richtung des mit den Borsten besetzten distalen Endes des Applikators 1, dann übt es Druck auf den gleich noch näher zu beschreibenden Kartuschenkolben 8 der Kartusche 3 aus.

Das Applikatorgehäuseoberteil 17 kann werkzeuglos (also im Regelfall mit bloßen Händen) ganz oder teilweise vom Applikatorhauptgehäuse abgenommen werden, um Zugang zu einem Kartuschenaufnahmeraum zu erhalten. Der Kartuschenaufnahmeraum ist bei diesem Ausführungsbeispiel der vom Applikatorgehäuseoberteil umgrenzte Innenraum. Generell lässt sich sagen, dass der Kartuschenaufnahmeraum vorzugsweise überwiegend oder besser mindestens im Wesentlichen durch das Applikatorgehäuseoberteil umgrenzt wird. Der Kartuschenaufnahmeraum besitzt stirnseitig einen Ausschnitt, durch den ein mit Borsten 4 besetzter Abschnitt der eigentlichen Kartusche (unter Verzicht auf einen als Rohr dienenden, zwischen dem Borstenbesatz und der eigentlichen Kartusche angeordneten Stiel) aus dem Kartuschenaufnahmeraum von innen nach außen herausragt. Vorzugsweise sind die Gestalt der Kartusche und des Kartuschenaufnahmeraums so aufeinander abgestimmt, dass die Kartusche bei geschlossenem Kartuschenaufnahmeraum klapperfrei in diesem gehalten wird. Für manche Anwendungsfälle ist es zweckmäßig, wenn der Kartuschenaufnahmeraum gegen Flüssigkeitszutritt von außen abgedichtet ist, hierauf wird später noch eingegangen.

Bei dem von Fig. 1 gezeigten Ausführungsbeispiel, welches nicht Teil der Erfindung ist, ist das Applikatorgehäuseoberteil 17 um eine nicht näher bezeichnete Achse in Richtung des Pfeils P1 schwenkbar an dem Applikatorhauptgehäuse 2 gehalten. Diese Schwenkbewegung wird im Normalbetrieb durch den Riegel 21 verhindert; sobald man Riegel 21 in Richtung des Pfeils P2 niedergedrückt hat, kann man jedoch das Applikatorgehäuseoberteil 17 in besagter Weise schwenken.

Alternativ, aber hier nicht gezeigt, lässt sich das Applikatorgehäuseoberteil 17 natürlich auch mit Hilfe eines Rastverschlusses oder eines Gewindes an dem Applikatorhauptgehäuse 2 befestigen, so dass das Applikatorgehäuseoberteil 17 beispielsweise durch eine Schraubbewegung vom Applikatorhauptgehäuse 2 gelöst werden, und schließlich durch eine Bewegung in Richtung parallel zur Applikatorlängsachse L abgezogen werden kann. Ähnlich kann ein Rastverschluss ausgeführt sein. Hier ist es zum Beispiel denkbar, dass das Applikatorhauptgehäuse 2 einen Ringflansch aufweist, der das Applikatorgehäuseoberteil 17 umgreift und in dem dieses verrastet werden kann - oder umgekehrt.

Die Fig. 1 zeigt, dass die besagte Schwenkbarkeit des Applikatorgehäuseoberteils 17 dazu dient, um eine Kartusche 3 in den Applikator 1 einsetzen zu können.

Sie besteht im Regelfall aus einem Kartuschenkörper 7 und einem darin dichtend und gleichzeitig beweglich eingesetzten Kartuschenkolben 8. Der Kartuschenkörper 7 besitzt vorzugsweise einen kreisrunden Querschnitt. Der maximale Kartuschenaußendurchmesser beträgt vorzugsweise 8 mm bis 20 mm. Die maximale Länge der Kartusche (ohne Borstenbesatz gemessen) in Richtung parallel zu Längsachse L beträgt vorzugsweise 5 mm bis 35 mm, besser nur 5 mm bis 20 mm. Für Single-Dose-Einsätze ist die Kartusche und/oder Blase zweckmäßigerweise so gestaltet, dass sie weniger als 10 ml, vorzugsweise weniger als 5 ml des zu applizierenden Fluids enthält. Hieran ist ihre äußere Größe angepasst.

Der Kartuschenkörper 7 ist auf der seinem distalen, mit Borsten besetzten Ende zugewandten Seite vorzugsweise mit einem Halsabschnitt versehen, dessen Außendurchmesser kleiner ist als der Außendurchmesser des Rests des Kartuschenkörpers 7. Der Halsabschnitt ragt mitsamt der an ihm befestigten Borsten bei gebrauchsfertig eingesetzter Kartusche durch den Ausschnitt des Kartuschenaufnahmeraums nach außen. Mithilfe der zwischen dem Halsabschnitt und dem Rest des Kartuschenkörpers 7 ausgebildeten Ringschulter stützt sich die Kartusche am Applikatorhauptgehäuse 2 bzw. im konkreten Ausführungsbeispiel am Applikatorgehäuseoberteil 17 gegen die vom Druckorgan 12 aufgebrachten, in Richtung parallel zur Längsachse L wirkenden Kräfte ab.

Die Kartusche 3, welche nicht Teil der Erfindung ist, enthält eine bestimmte Menge eines flüssigen Kartuscheninhalts 6, der hier durch die Punktierung angedeutet ist. Bei dem Kartuscheninhalt 6 handelt es sich normalerweise um ein dünnflüssiges Fluid, das als Kosmetikum und/oder als Pharmazeutikum appliziert werden soll. Vorzugsweise, aber nicht ausschließlich, wird unter dem Begriff "dünnflüssig" ein Fluid verstanden, das eine Viskosität von weniger als 100 mPa x s aufweist.

Die Kartusche 3, welche nicht Teil der Erfindung ist, zeichnet sich durch ihren sehr einfachen Aufbau aus, aufgrund dessen ist sie sehr rationell aus Kunststoff in einem oder wenigen Arbeitsschritten herstellbar.

Vorzugsweise wird der Applikator 1 so verwendet, dass er mit EinwegKartuschen 3 betrieben wird. Für viele, insbesondere medizinische Anwendungen ist es besonders vorteilhaft, wenn die Einweg-Kartusche als sog. "single-dose" Kartusche verwendet wird, d. h., die bis zu ihrem Einsatz vorzugsweise steril verpackte Kartusche enthält im Wesentlichen nur so viel des zu applizierenden Fluids, wie für eine einmalige Anwendung benötigt wird. Danach wird die Kartusche mitsamt dem daran befestigten Borstenbesatz verworfen. Auf diese Art können auch hohe Hygieneanforderungen kostengünstig mit einem maximalen Bedienungskomfort durch den nicht als Einwegteil konzipierten, motorisch betriebenen Applikator erfüllt werden.

Die von Fig. 1 gezeigte Kartusche, welche nicht Teil der Erfindung ist, zeichnet sich dadurch aus, dass der Kartuscheninhalt 6 von Anfang an unmittelbar mit dem Kartuschenkörper 7 in Kontakt kommt, d. h. der Kartuscheninhalt 6 wird bei der Herstellung der Kartusche 3 direkt in die Kartusche 3 abgefüllt. Im Regelfall wird erst danach der Kartuschenkolben 8 eingesetzt, um die große, bequem zu benutzende Abfüllöffnung zu verschließen. Zu diesem Zweck ist der Kartuschenkolben 8 so ausgeführt, dass er mit der nötigen Vorspannung gegenüber der Innenumfangsfläche des Kartuschenkörpers 7 anliegt. Um dies zu erreichen, ist der Kartuschenkolben 8 vorzugsweise mit einem Ringkragen 22 ausgerüstet, den man ebenfalls in Figur 1 sieht. Im unbelasteten Zustand verläuft dieser Ringkragen 22 bezogen auf die Längsachse L vorzugsweise leicht schräg-kegelig nach außen. In dem Moment, in dem der Kartuschenkolben 8 erstmals in den Kartuschenkörper 7 eingeschoben wird, wird der zu diesem Zeitpunkt kegelig nach außen geneigt verlaufende Ringkragen 22 zusammengedrückt und liegt hierdurch mit der erforderlichen Vorspannung gegen die Innenumfangsfläche des Kartuschenkörpers 7 an. Hierdurch wird im Regelfall eine hinreichende Abdichtung erreicht, ohne dass z. B. ein zusätzlicher Dichtring in Gestalt einer separaten Schnurdichtung erforderlich wird.

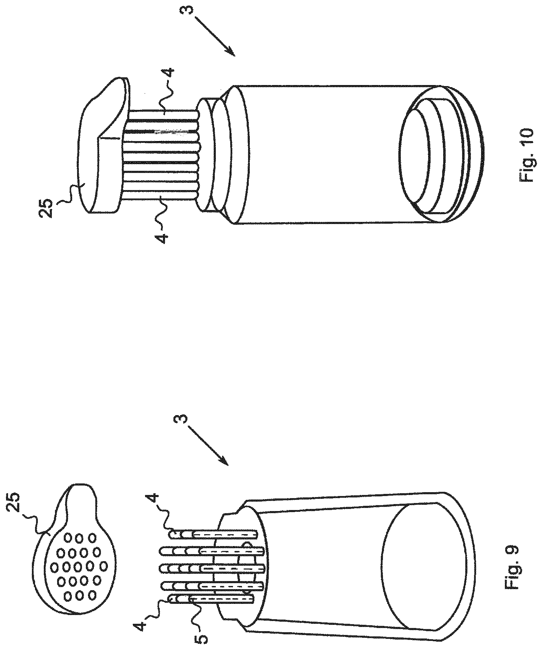

Es ist in vielen Fällen zweckmäßig, wenn der Kartuschenkolben 8 die Gestalt des Halsabschnitts des Kartuschenkörpers 7 aufgreift, d. h. einen innen vorspringenden Abschnitt mit einem verringerten Außendurchmesser aufweist, der in den von dem Hals gebildeten Abschnitt mit verringertem Durchmesser einfahren kann und so für eine verbesserte bzw. vollständige Entleerung der Kartusche sorgt, vgl. auch Fig. 10.

Der Kartuschenkörper 7 ist einstückig mit den Borsten 4 verbunden, d. h., die Borsten 4 können nicht zerstörungsfrei vom Kartuschenkörper 7 getrennt werden. Der Kartuschenkörper und die Borsten bilden eine gemeinsam zu handhabende Nachfülleinheit.

Die Lebensdauer des Applikators als Ganzem ist nicht durch die Lebensdauer der Borsten oder durch die unter Verkeimungsgesichtspunkten zulässige Gebrauchszeit der Borsten beschränkt.

Bei dem für Figur 1 gezeigten Ausführungsbeispiel der Kartusche 3, welches nicht Teil der Erfindung ist, ist jede der Borsten 4 innen hohl und bildet somit einen sich durch die Borste 4 hindurch erstreckenden Borstenkanal 5 aus. Das ist eine für eine Anzahl von Anwendungen vorteilhafte Ausführungsform, da eine optimale Verteilung des zu applizierenden Materials erreicht wird, das u. U. sehr teuer und daher mit maximaler Sparsamkeit zu verwenden ist. In anderen Fällen, in denen der Schwerpunkt weniger auf dem sparsamen Einsatz des aufzutragenden Fluids liegt als auf der möglichst preisgünstigen Herstellung der Kartuschen, sind letztere vorzugsweise nur mit einer einzigen hohlen Borste versehen.

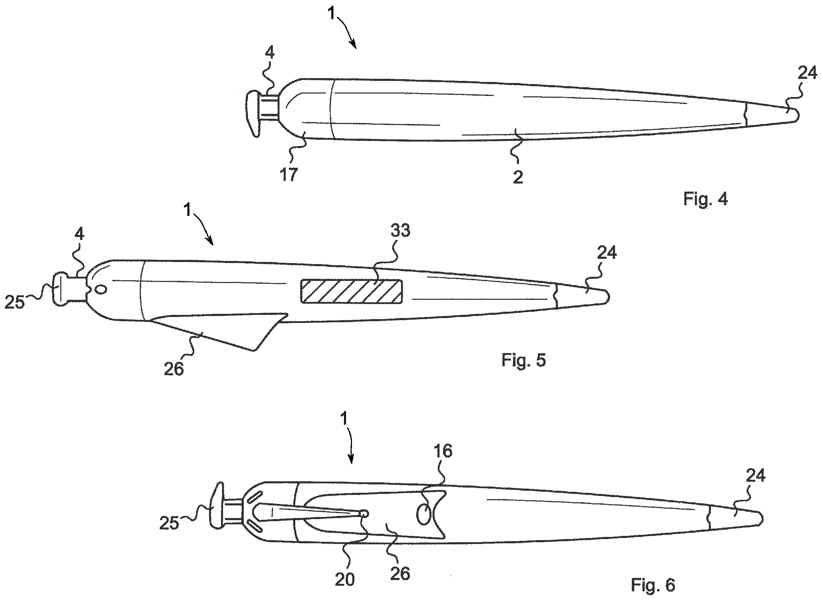

Der Borstenkanal 5 erstreckt sich nicht nur durch die Borsten 4, sondern auch durch die Borstenbasisschicht 10 hindurch, die sich auf der Innenseite der distalen Stirnwand des Kartuschenkörpers 7 befindet. Diese Borstenbasisschicht 10 besteht aus dem gleichen Material wie die Borsten, ist einstückig mit den Borsten 4 verbunden und durchgreift die distale Stirnseite des Kartuschenkörpers 7, was im Regelfall seine Ursache in dem gleich noch näher geschilderten Herstellungsverfahren hat. Die Borstenkanäle 5 sind hier jeweils nur durch eine dünne schwarze Volllinie angedeutet, die sich mitten durch die Borsten 4 und die Borstenbasisschicht 10 hindurch erstreckt. Besonders vorteilhaft ist es, wenn man die Borstenkanäle 5 im Hinblick auf ihren Durchmesser so auf den Kartuscheninhalt 6 abstimmt, dass durch die Kapillarwirkung der Borstenkanäle der Kartuscheninhalt 6 am Austritt durch die Borstenkanäle gehindert wird, solange der Kartuscheninhalt 6 nicht unter Druck gesetzt wird. Eine solche Ausgestaltung ist allerdings nicht zwingend. Alternativ, nämlich insbesondere dann, wenn die Borstenkanäle 5 einen größeren Durchmesser haben, können sie auch durch eine entsprechend ausgestaltete Kappe 25 verschlossen werden, vgl. Fig. 4 bis 6, 9, 10 und 14.

Um den Kartuscheninhalt 6 im Zuge der Applikation nach außen auszugeben, betätigt der Anwender den Schalter 16. Hierdurch wird der Motor aktiviert, der dann den bereits oben beschriebenen Körper in Richtung auf den Kartuschenkolben 8 zubewegt und schließlich gemeinsam mit dem Kartuschenkolben 8 in Richtung des distalen Endes des Applikators 1 fährt. Hierdurch wird das dem Kartuscheninhalt 6 zur Verfügung stehende Volumen verringert. Es baut sich im Kartuscheninhalt 6 ein Druck auf, der einen Teil des Kartuscheninhalts 6 durch die Borstenkanäle 5 hindurch nach außen ausgibt. Der Kartuscheninhalt 6 tritt also an der Spitze der Borsten 4 aus.

Es leuchtet ein, dass man aufgrund dessen den Kartuscheninhalt 6 besonders gut und in hohem Maße verlustfrei auf behaarte Hautpartien auftragen kann, wo mit den herkömmlichen Mitteln - etwa beim herkömmlichen Auftrag mit Hilfe eines Wattestäbchens - viel Substanz verloren geht und zudem die Haare unnötig verklebt werden.

Unter einer Borste im Sinne der Erfindung wird - technisch ausgedrückt - ein einseitig eingespannter Biegestab verstanden, dessen maximaler Durchmesser Dmax oberhalb der Verrundung, d. h. der Borstenwurzel, mit der die Borste in den sie tragenden Untergrund übergehen mag, mindestens um den Faktor 5, besser mindestens um den Faktor 10 kleiner ist als die maximale Länge Lmax der Borste in Richtung parallel zu ihrer Borstenlängsachse (Mittellängsachse) BL. Idealerweise ist die Borste so flexibel, dass sich ihre Spitze - bezogen auf den Verlauf ihrer Mittellängsachse BL in unbelastetem Zustand - durch die bei der bestimmungsgemäßen Applikation auftretenden Kräfte reversibel um mindestens die Strecke AL senkrecht zur Mitellängsachse BL auslenken lässt. Idealerweise ist jede Borste mit einer in Bezug auf die Borstenlängsachse BL kegeligen oder schräg verlaufenden Mantelfläche ausgestattet, die bevorzugt einen Winkel α mit der Borstenlängsachse einschließt, der zwischen 0,5° und 2,5° beträgt. Dies gilt nicht nur für das hier erörterte Ausführungsbeispiel, sondern für alle Ausführungsbeispiele.

Der Borstenbesatz wird im Regelfall nicht getuftet ausgeführt, d. h. die Borsten sind nicht büschelweise in den sie tragenden Untergrund implantiert, sondern jede einzelne Borste weist schon im Bereich ihres Borstenfußes rundum einen Abstand von den benachbarten Borsten auf. Die Borsten bilden im Regelfall auch nicht nur einen Kamm aus entlang einer geraden Linie hintereinander aufgestellten Borsten, sondern sind vorzugsweise in mehreren Reihen hintereinander aufgestellt, die jeweils eine in sich geschlossene Kreisringlinie bilden. Dabei sind die mehreren Kreisringlinien meist konzentrisch ineinander angeordnet. Idealerweise sind nicht nur einzelne Borsten vorgesehen, sondern ein Borstenfeld aus 12 bis 30 Borsten. Diese besitzen vorzugsweise jeweils eine Mittellängsachse, die parallel zur Applikatorlängsachse L verläuft. Die Borsten sind vorzugsweise aus einem weichelastischen Material gefertigt.

Um den hier gezeigten Borstenbesatz herzustellen, der sich dadurch auszeichnet, dass seine Borstenbasisschicht 10 mit der Innenoberfläche der distalen Stirnseite des Kartuschenkörpers 7 verschweißt ist, gibt es unterschiedliche Möglichkeiten.

Die erste Möglichkeit ist die, dass in einem ersten Spritzgussschritt der Kartuschenkörper 7 spritzgegossen wird, wobei der Kartuschenkörper 7 auf der Seite, auf der später der Kartuschenkolben 8 eingesetzt wird, zunächst offen bleibt. Die distale Stirnseite des Kartuschenkörpers 7 wird bereits in diesem Schritt mit Löchern versehen, die durch entsprechende Stifte an einer der Formhälften erzeugt werden. Als nächstes wird die Formhälfte, die die besagten Stifte trägt, abgenommen. Stattdessen wird eine andere Formhälfte aufgesetzt, die ihrerseits Stifte trägt, die schlanker sind und die die im Kartuschenkörper 7 von den vorher genannten Stiften hinterlassenen Löcher zumindest in etwa mittig durchgreifen und gegen den Kern, der nach wie vor in dem Kartuschenkörper 7 angeordnet ist, abdichten. Dann wird, im Regelfall von der Innenseite des Kartuschenkörpers 7 her, eine zweite Kunststoffmasse eingespritzt, die die Borsten 4 bildet. Die zweite Kunststoffmasse bildet dann die Borstenbasisschicht 10 an der Innenoberfläche der distalen Stirnseite des Kartuschenkörpers 7 aus und fließt durch die Ringspalte, die zwischen den Löchern in der Stirnseite des Kartuschenkörpers 7 und den durch sie hindurchragenden Stiften der zweiten Formhälfte frei bleibt, in die Borsten bildenden Kavitäten in der zweiten Formhälfte ein.

Sobald die zweite Kunststoffmasse erstarrt ist, wird die zweite Formhälfte mit ihren schlanken Stiften abgezogen. Die schlanken Stifte hinterlassen in den Borsten 4 jeweils einen Borstenkanal 5. Schließlich wird der Kartuschenkörper 7 vollständig ausgeformt, indem er von dem Kern, der derzeit noch in ihm verweilt, abgestoßen wird.

Eine alternative Möglichkeit, um die von Figur 1 gezeigten Borsten 4 mit einem etwa mittigen Borstenkanal 5 auszubilden, funktioniert wie folgt:
Es wird zunächst der Kartuschenkörper 7 (ohne Kartuschenkolben 8, der später eingesetzt wird) gespritzt, und zwar so, dass der Kartuschenkörper 7 zunächst eine durchgehend geschlossene Stirnseite aufweist. Dann wird die die Außenseite der Stirnseite des Kartuschenkörpers 7 begrenzende Formhälfte abgenommen und durch eine andere Formhälfte ersetzt, die mit gut belüfteten, Borsten bildenden Kavitäten ausgestattet ist. Jetzt wird wiederum von der Seite des Formkerns aus, der sich in dem Kartuschenkörper 7 befindet, die zweite Kunststoffmasse eingespritzt. Diese staut sich zumindest einen winzigen Augenblick vor der noch in sich geschlossenen Stirnwand des Kartuschenkörpers 7, so dass hier ein hoher Druck anliegt. Unter dem Einfluss dieses hohen Drucks und eventuell auch der hohen Temperatur der soeben eingespritzten zweiten Kunststoffmasse punktiert die zweite Kunststoffmasse die Stirnseite des Kartuschenkörpers 7 lokal - nämlich überall dort, wo die Stirnseite des Kartuschenkörpers 7 "hohl" liegt, also über einer auf der Außenseite angeordneten, Borsten bildenden Kavität. Sobald der Durchbruch erfolgt ist, schießt die zweite Kunststoffmasse durch die von ihr selbst im Kartuschenkörper 7 ausgebildeten Öffnungen in die borstenformenden Kanäle ein. Bei entsprechender Kühlung der Formhälfte mit den die Borsten bildenden Kanälen erstarrt die zweite Kunststoffmasse zunächst im Randbereich der Form, während jede Borste noch einen Kern aus flüssiger Kunststoffmasse aufweist. Lässt man hier nun zum richtigen Augenblick Druckluft ein, dann treibt diese den noch schmelzflüssigen Kern jeder Borste aus und schafft so, von der Innenseite der Kartusche her kommend, einen Borstenkanal 5 in jeder Borste 4. Ein solches Verfahren ist besonders rationell, da der Formaufwand gering gehalten wird. Es ist lediglich Fingerspitzengefühl dafür erforderlich, in welchem Augenblick Luft eingeblasen werden muss, um den noch schmelzflüssigen Borstenkern auszutreiben. Dieses Fingerspitzengefühl kann und muss der Fachmann durch Versuche erwerben, denn wie der richtige Zeitpunkt zu steuern ist, hängt von verschiedenen Randparametern des Einzelfalls ab und kann daher nicht allgemeingültig angegeben werden.

Es wird auch eigenständiger Schutz für dieses Verfahren beansprucht.

Zu betonen ist, dass nicht zuletzt die oben geschilderten Vorgaben für den Borstenbesatz und dessen Herstellung für alle Ausführungsbeispiele vorteilhaft sind.

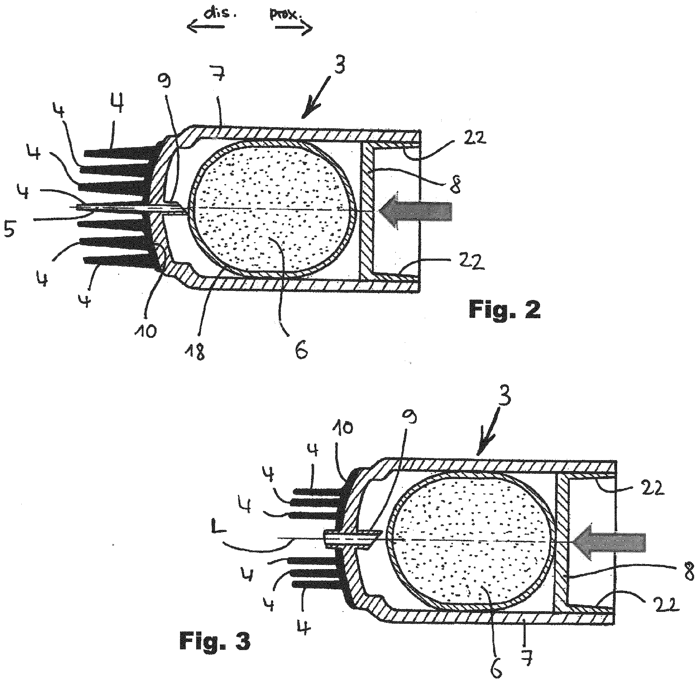

Eine erste Ausgestaltungsmöglichkeit der Kartusche 3 für einen erfindungsgemäßen Applikator 1 zeigt die Fig. 2.

Auch diese Kartusche besteht aus einem Kartuschenkörper 7 und einem Kartuschenkolben 8, der vorzugsweise so ausgestaltet ist, wie das oben für das erste Ausführungsbeispiel beschrieben wurde. Insbesondere liegt der Kartuschenkolben 8 dichtend gegen die Innenoberfläche des Kartuschenkörpers 7 an, so, wie oben geschildert.

Anders als beim Ausführungsbeispiel der Fig. 1 wird der Kartuscheninhalt 6 hier jedoch bei der Herstellung nicht unmittelbar in die Kartusche 3 abgefüllt, so dass er sofort mit der Innenoberfläche des Kartuschenkörpers 7 und/oder des Kartuschenkolbens 8 in Berührung käme. Stattdessen wird der Kartuscheninhalt 6 in eine hermetisch geschlossene Blase 18 abgefüllt, bei der es sich vorzugsweise um eine Blase aus einer Folie handelt oder um eine Hülle aus Gelee, nachfolgend pauschal "Blase" genannt.

Anders als die Kartusche 3 des Ausführungsbeispiels der Fig. 1, ist diese Kartusche 3 vorzugsweise im Bereich ihrer distalen Stirnseite innenseitig mit mindestens einem - und bevorzugt nur einem - Dorn 9 ausgestattet, der idealerweise eine Passage nach außen bildet. Sobald der Kolben 8 das erste Mal zum distalen Ende der Kartusche 3 hin verschoben wird, presst er die Blase 18 gegen den Dorn 9, der diese schließlich punktiert. Auf diese Art und Weise kann dann der Kartuscheninhalt 6 über die hier vom Dorn 9 gebildete Passage nach außen ausgegeben werden. Die Befüllung der Kartusche 3 mit Hilfe einer solchen Blase 18 hat den großen Vorteil, dass bis zum endgültigen Anbrechen der Kartusche 3 sichergestellt werden kann, dass der Kartuscheninhalt 6 vor jeglichem Kontakt mit der Umwelt geschützt ist. Dies ist insbesondere bei pharmazeutischen Anwendungen von großer Bedeutung, aber auch bei kosmetischen Anwendungen. Auf diese Art und Weise kann nicht nur Keimfreiheit garantiert werden, sondern im Regelfall auch ein wesentlich langsameres Verderben des Kartuscheninhalts 6, der weitestgehend vor Luftzutritt bewahrt werden kann - die nie ganz zu vermeidbaren Diffusionsvorgänge natürlich ausgenommen.

Der Besatz aus den Borsten 4, der auch bei diesem Ausführungsbeispiel einstückig, d. h. untrennbar, mit dem Kartuschenkörper 7 verbunden ist, zeichnet sich hier durch eine optionale andere Art der Herstellung aus.

Auch hier ist eine Borstenbasisschicht 10 vorgesehen, die nun allerdings mit der Außenoberfläche der distalen Stirnseite des Kartuschenkörpers 7 verschweißt ist. Von dieser Borstenbasisschicht 10 aus gehen die einzelnen Borsten 4 ab.

Vorzugsweise ist hier nur eine der Borsten 4 mit einem Borstenkanal 5 versehen, nämlich idealerweise die zentrale Borste, die mit dem Dorn 9 kommuniziert. Diese Borste ist vorzugsweise um mindestens 30 % dicker als die anderen Borsten.

Hergestellt worden ist dieser Borstenbesatz wie folgt:
Zunächst ist hier ein Kartuschenkörper 7 mit einer vollständig geschlossenen distalen Stirnseite gespritzt worden. Die einzige Ausnahme ist der Innenbereich des Dorns 9, der von vornherein mit Hilfe eines entsprechenden Dorns am Werkzeug freigehalten worden ist. Nach dem Spritzen des Kartuschenkörpers 7 ist der Formteil abgenommen worden, der die äußere distale Stirnseite des Kartuschenkörpers 7 abgebildet hat. Stattdessen ist hier nun eine Formhälfte aufgesetzt worden, die Borsten bildende Kavitäten aufweist und die im Bereich der zentralen Borste den im Bereich des Dorns 9 durch die Stirnseite des Kartuschenkörpers 7 hindurchragenden dornartigen Schieber aufnimmt, der vorzugsweise zu dem Formkern gehört, der im Inneren des Kartuschenkörpers steckt. Sodann wurde in einem zweiten Schritt eine zweite Kunststoffmasse eingespritzt, die die Borsten bildende Kavitäten ausfüllt und die Borstenbasisschicht 10 bildet. Die heiße zweite Kunststoffmasse verklebt oder verschweißt mit der Oberfläche der bereits abgekühlten Kunststoffmasse, die den Kartuschenkörper 7 bildet, so dass es zu der besagten Einstückigkeit kommt. Danach wird ausgeformt.

Auch bei diesem Ausführungsbeispiel wird für die Borsten 4, genauso wie beim ersten Ausführungsbeispiel, vorzugsweise ein Weichelastomer eingesetzt.

Bei diesem Ausführungsbeispiel ist noch festzuhalten, dass es Vorteile hat, wenn man sich darauf beschränkt, nur die zentrale Borste mit einem Borstenkanal 5 zu versehen und dadurch nur über die zentrale Borste den Kartuscheninhalt auszugeben. Auf diese Art und Weise wird nämlich verhindert, dass der ausgegebene Kartuscheninhalt 6 vorzeitig seitlich aus dem Bereich des Borstenfeldes entweicht und dann sozusagen nicht mehr zum Einmassieren zur Verfügung steht.

Aufgrund dessen sieht ein hier nicht figürlich dargestelltes, aber bevorzugtes Ausführungsbeispiel, welches aber nicht Teil der Erfindung ist, so aus, dass das erste und das zweite Ausführungsbeispiel dahingehend kombiniert werden, dass in der Kartusche 3 keine Blase vorgesehen wird, sondern der Kartuscheninhalt direkt in die Kartusche 3 eingefüllt wird, wobei aber die Ausgabe dann, anders als im ersten Ausführungsbeispiel, nur über eine zentrale Borste erfolgt, so, wie im zweiten Ausführungsbeispiel. Dies hat bei Verzicht auf eine Blase 18 zugleich den Vorteil, dass sich der Kartuscheninhalt besser abdichten lässt - eine auf die Borsten aufgesetzte Kappe, wie sie später noch erläutert wird, die nur eine der Borsten abdichten muss, ist leichter zu realisieren als eine Kappe, die sämtliche Borsten abdichtet.

Ein weiteres Ausführungsbeispiel einer Kartusche für den erfindungsgemäßen Applikator zeigt die Figur 3.

Die Kartusche entspricht hier fast vollständig dem zweiten Ausführungsbeispiel, weshalb das dort für die Kartusche und das Herstellungsverfahren der Borsten Gesagte sinngemäß auch für diese Kartusche gilt, soweit sich aus den nachfolgend ausdrücklich geschilderten Unterschieden nicht etwas anderes ergibt.

Der Unterschied zwischen dem zweiten Ausführungsbeispiel und dem dritten Ausführungsbeispiel liegt darin, dass hier der Dorn 9 nicht mit einer Borste verbunden ist, sondern, wie von Figur 3 gezeigt, innerhalb des Borstenfeldes, das im Bereich des Dorns 9 eine Aussparung hat, nach außen endet. Auf diese Art und Weise kann der Kartuscheninhalt 6 in einen von den sehr eng stehenden Borsten umgrenzten, quasi abgeschlossenen Bereich ausgegeben werden. Vorzugsweise bildet der Dorn 9 einen Fortsatz aus, der nach außen übersteht. Der Fortsatz bildet einen wenig verformbaren Rohrflansch, der sich einfach mit Hilfe einer nachträglich montierten Kappe abdichten lässt.

Beim unmittelbaren Vergleich zwischen den Fig. 2 und 3 sieht man noch, wie der lichte Innendurchmesser des Dorns 9 variiert werden kann, um auf diese Art und Weise eine Anpassung an die Viskosität des Kartuscheninhalts 6 vorzunehmen. Denn der lichte Querschnitt des Dorns 9 darf nicht zu groß sein. Nur so kann verhindert werden, dass der Kartuscheninhalt 6 nach dem Anbruch der Blase 18 unbeabsichtigt nach außen ausläuft. Hier gilt wieder, dass der lichte Querschnitt vorzugsweise so dimensioniert ist, dass die Kapillarwirkung des Dorns 9 den Kartuscheninhalt 6 daran hindert, nach außen auszulaufen, solange nicht durch den Kartuschenkolben 8 Druck auf den Kartuscheninhalt 6 ausgeübt wird.

Die Figuren 4 bis 6 zeigen ein besonderes, nämlich nicht nur eleganter, sondern auch funktioneller gestaltetes Ausführungsbeispiel für ein Applikatorhauptgehäuse 2 mit einem Applikatorgehäuseoberteil 17.

Vom Grundsatz her enstpricht auch dieses Ausführungsbeispiel vollständig dem bereits geschilderten Ausführungsbeispiel. Daher gilt das oben Gesagte auch für dieses Ausführungsbeispiel, soweit sich aus den nachfolgenden Schilderungen nicht ausdrücklich etwas anderes ergibt.

Im Unterschied zu dem ersten Ausführungsbeispiel ist das Applikatorhauptgehäuse hier so gestaltet, dass es zum proximalen, dem Borstenbesatz abgewandten Ende hin immer schlanker wird. Es endet vorzugsweise in einer Elastomerspitze 24, die im Idealfall weniger als 50 Prozent des maximalen Durchmessers des Applikators 1 ausmacht. Diese Elastomerspitze 24 kann zum Eimassieren des zuvor mit Hilfe der Borsten 4 aufgetragenen Kartuscheninhalts 6 dienen.

Gut zu erkennen ist hier das Applikatorgehäuseoberteil 17. Dies ist hier vorzugsweise als Schraub-Oberteil ausgebildet. Ansonsten wird die Kartusche 3 aber so aufgenommen, wie das für das erste Ausführungsbeispiel geschildert worden ist. Wie man sieht, kann das Applikatorhauptgehäuse 2 sehr gut als Werbe- und oder Informationsträger dienen, indem es durch Bedrucken oder Bekleben mit einem entsprechenden Label 33 versehen wird.

Wie hier gut zu erkennen ist, werden die Borsten 4 hier bis zum Anbruch durch eine Kappe 25 geschützt und verschlossen.

Anders ausgeführt als bei dem ersten Ausführungsbeispiel ist hier der Schalter 16. Der Schalter 16 ist hier vorzugsweise in einem in radialer Richtung zur Längsachse L deutlich nach außen über die umgebende Oberfläche des Applikatorhauptteils hinausragenden Vorsprung 26 angeordnet. Dieser schafft nicht nur zusätzlich Platz für den Schalter 16 und die ihm eventuell zugeordnete elektronische Platine, sondern dient zugleich bei der Anwendung auch als Daumenstopper. Durch diesen wird sichergestellt, dass der Applikator ergonomisch korrekt gehalten wird und dabei der Schalter 16 leicht bedient werden kann.

Der besagte Vorsprung 26 beherbergt vorzugsweise gleichzeitig auch eine Lampe 20, im Idealfall in Gestalt einer LED. Hiermit kann die Partie, auf die appliziert werden soll, beleuchtet werden. Dies wird insbesondere dann als angenehm empfunden, wenn der Applikator 1 dazu verwendet wird, um einen Behandler einer anderen Person eine Applikation verabreichen zu lassen.

Idealerweise ist die Außenoberfläche des Applikatorhauptgehäuses 2 und des Applikatorgehäuseoberteils 17 sowie eines eventuellen Batteriefachdeckels 14 metallisiert. Das gewährleistet, dass sich der Applikator besonders gut reinigen und gegebenenfalls auch mit einem Desinfektionsmittel behandeln lässt, wobei eventuell verbliebene Verunreinigungen durch die Metallisierung sofort augenfällig werden.

Die Figuren 9 und 10 zeigen noch einmal die vom ersten Ausführungsbeispiel verwendete Kartusche 3 in Einzelansicht.

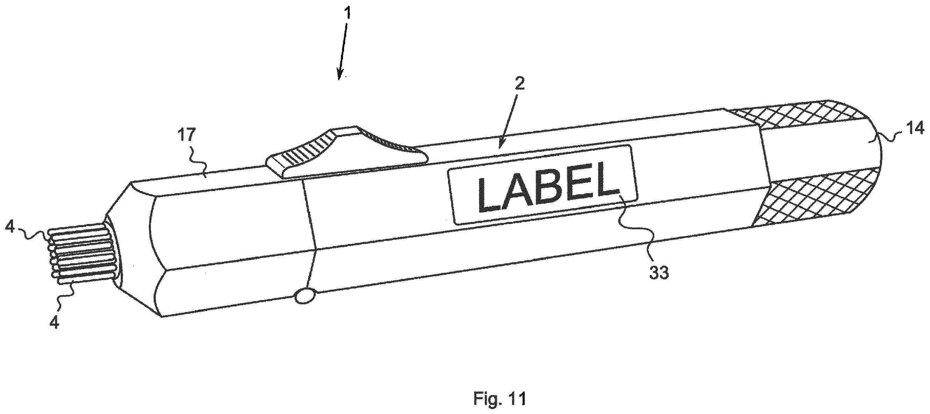

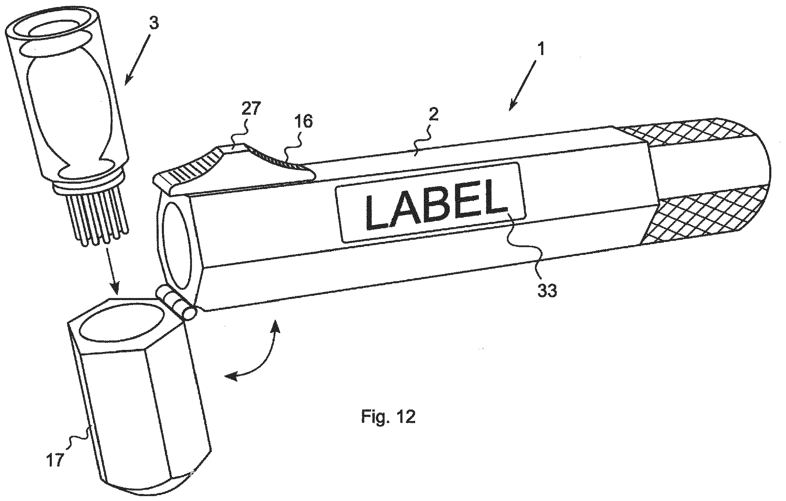

Die Figuren 11 und 12 zeigen noch ein weiteres Ausführungsbeispiel, das nahezu vollständig mit dem ersten Ausführungsbeispiel identisch ist, so dass das dort Gesagte auch für dieses Ausführungsbeispiel gilt, sofern sich nicht nachfolgend aus den explizit genannten Unterschieden etwas anderes ergibt.

Man sieht anhand der Figuren 11 und 12 noch einmal recht schön, wie hier der Applikator 1 in ein Applikatorhauptgehäuse 2, ein Applikatorgehäuseoberteil 17 und vorzugsweise auch einen Batteriefachdeckel 14 unterteilt ist. Wie man auch hier wieder sieht, kann das Applikatorhauptgehäuse 2 sehr gut als Werbe- und/oder Informationsträger dienen, indem es durch Bedrucken oder Bekleben mit einem entsprechenden Label 33 versehen wird.

Wie schon bei dem ersten Ausführungsbeispiel ist das Applikatorgehäuseoberteil 17 einseitig schwenkbar an dem Applikatorhauptgehäuse 2 angelenkt, so dass das Applikatorgehäuseoberteil 17 ähnlich wie der Lauf einer Schrotflinte abgeklappt werden kann, um eine (neue) Kartusche 3 einzusetzen.

Wie man anhand der Fig. 12 sieht, kommt hier eine Kartusche zum Einsatz, wie sie im Rahmen des Ausführungsbeispiels der Fig. 2, 3 beschrieben worden ist. Alternativ kann aber auch eine Kartusche eingesetzt werden, wie sie im Rahmen des zweiten Ausführungsbeispiels beschrieben worden ist.

Wie man anhand der Fig. 12 gut erkennen kann, kommt eine spezielle Anordnung für den Schalter 16 zum Einsatz. Der Schalter 16 ist in einem Schiebeelement 27 untergebracht, das gleichzeitig zur Verriegelung des abklappbaren Applikatorgehäuseoberteils 17 dient.

Für alle hier beschriebenen Ausführungsbeispiele kann es vorteilhaft sein, wenn das gesamte Applikatorgehäuse oder zumindest das Applikatorgehäuse im Bereich, in dem es von der Kartusche und ihrem Borstenbesatz durchgriffen wird, abgedichtet ausgeführt ist, so dass das distale Ende des Applikators 1 mit dem Borstenbesatz der Kartusche beispielsweise unter einen Wasserstrahl gehalten und ausgewaschen werden kann, ohne dass das hierfür verwendete Wasser in den Applikator 1 eindringt. Um dies zu erreichen, kann es zweckmäßig sein, beispielsweise an der Kartusche und/oder an dem Applikatorgehäuseoberteil 17 in dem Bereich, in dem die Kartusche 3 das Applikatorgehäuseoberteil 17 durchgreift, eine Dichtung D vorzusehen, die Wassereintritt durch den Spalt zwischen dem Applikatorgehäuseoberteil 17 und dem entsprechenden Bereich der Kartusche 3 verhindert. Eine solche Dichtung D kann z. B. so aussehen, wie das die Fig. 1a zeigt, bei der es sich um eine Ausschnittvergrößerung des in Fig. 1 durch einen Kreis markierten Bereichs handelt. Rund um den Ausschnitt des Applikatorgehäuseoberteils, durch den der Halsabschnitt der Kartusche mit dem daran befestigten Borstenfeld hindurchragt, ist eine lippenartige Dichtung D aus weichelastischem Material angespritzt.

Besonders zweckmäßig ist es natürlich, wenn auch die Trennfuge zwischen dem Applikatorgehäuseoberteil 17 und dem Applikatorhauptgehäuse 2 mit einer Dichtung versehen ist, die beispielsweise verhindert, dass zum Spülen verwendetes Wasser hier eindringt.

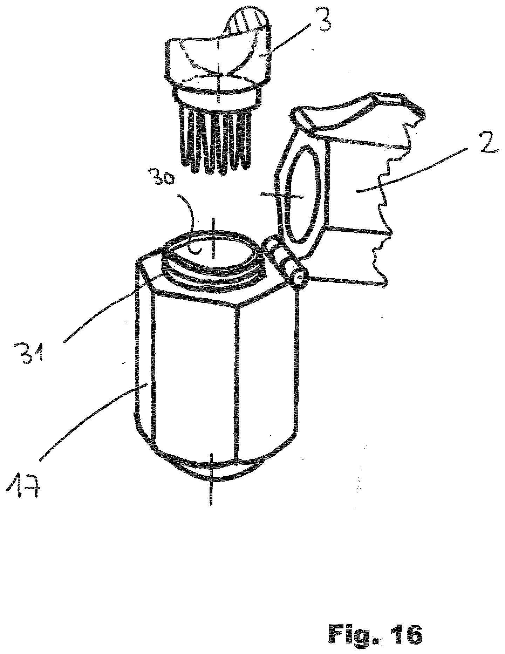

Eine solche Dichtung lässt sich besonders einfach dadurch realisieren, dass beispielsweise das Applikatorgehäuseoberteil 17 einen Ringkragen 30 zur Einführung in das Applikatorhauptgehäuse aufweist, der eine Schnur- oder Lippendichtung 31 trägt, die sich in dem Moment, in dem der besagte Ringkragen 30 von dem Applikatorhauptgehäuse 2 aufgenommen wird, gegen dessen Innenoberfläche anlegt, vgl. Fig. 16.

Für manche Anwendungsfälle ist es besonders bevorzugt, wenn der Applikator bzw. das Applikatorhauptgehäuse 2 nicht nur die Batterie und den Motor und das Druckorgan 12 beinhaltet, sondern beispielsweise zusätzlich noch ein Vibrationselement, welches Schwingungen erzeugt, vorzugsweise solche, die im Wesentlichen in Richtung quer zur Längsachse L verlaufen. Der Applikator 1 kann dann gleich dazu verwendet werden, um mit Hilfe von schnellen, aber kurzhubigen Bewegungen den Kartuscheninhalt 6, der nach außen ausgegeben wurde, einzumassieren und vorzugsweise bei dieser Gelegenheit auch die zu behandelnde Hautpartie zu stimulieren.

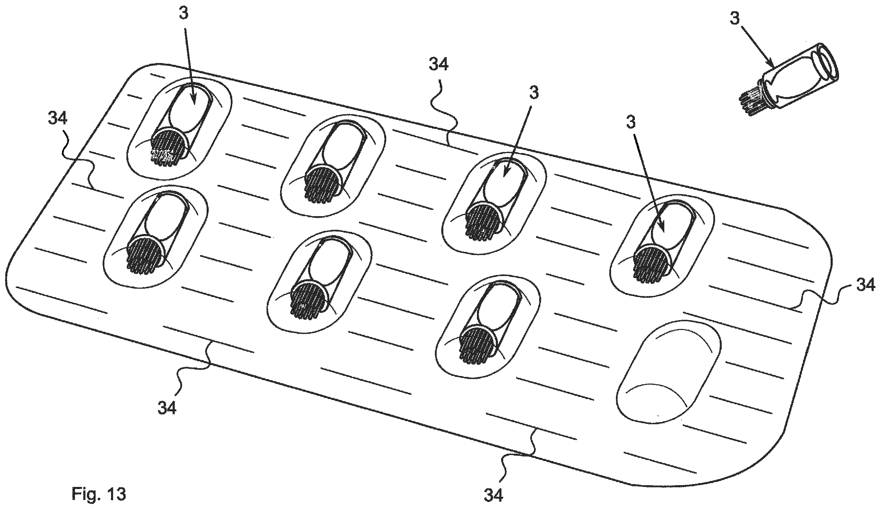

Gerade für Anwendungsfälle, bei denen der Kartuscheninhalt 6 besonders schutzbedürftig ist, bietet es sich an, den erfindungsgemäßen Applikator 1 und die für seinen Betrieb erforderliche Kartusche 3 dadurch zu einem System zu kombinieren, dass die Kartusche 3 in sogenannten Blister-Packs zusammengefasst und gelagert werden. Diese Blister-Packs kennt man von Tablettenpackungen her. Es handelt sich im Regelfall um einen Kunststoffträger, der einzelne Vertiefungen aufweist, die jeweils vorzugsweise eine Kartusche 3 enthalten. Nach dem Befüllen dieses Kunststoffträgers mit den Kartuschen wird die Rückseite des Kunststoffträgers vorzugsweise durch eine dünne Metallfolie, die aufgeschweißt wird, versiegelt. Um eine Kartusche zum Nachladen des Applikators 1 zu entnehmen, wird diese durch die Metallfolie hindurch nach außen gedrückt. Die anderen Kartuschen 3 bleiben noch geschützt durch die Metallfolie in dem Kunststoffträger in ihrer Vertiefung bevorratet. Die Fig. 13 zeigt dies. Anzumerken ist noch, dass der Kunststoffträger ebenfalls gut als Werbe und/oder Informationsfläche diesen kann, indem seine freien Zwischenräume mit einer Beschriftung 34 versehen werden, wie sie in Fig. 13 jeweils durch eine Schar schwarzer Linien angedeutet wird.

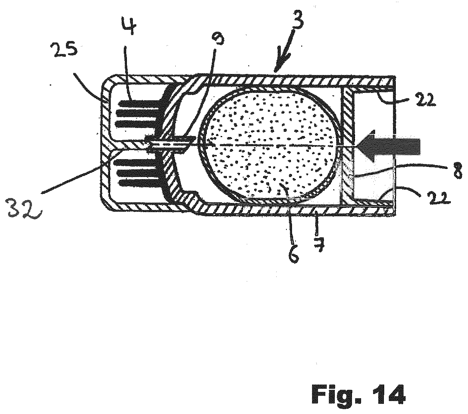

Anzumerken ist noch, dass das erfindungsgemäße System, wie schon vermerkt, auch gut dazu verwendet werden kann, um Skincare Produkte, Salben und Cremes auf die unbehaarte Haut aufzutragen. Soweit diese Produkte höherviskos sind (wie meist), wird dem durch entsprechende Vergrößerung des lichten Querschnitts der innen hohlen Borste (4) Rechnung getragen. Zu diesem Zwecke sind solche Lösungen, bei denen nur eine zentrale Borste innen hohl ist, besonders bevorzugt. Die Fig. 14 zeigt eine Kappe 25, wie sie zum Abdichten zum Einsatz kommen kann. Die Kappe lässt sich auf der Kartusche befestigen, insbesondere aufstecken oder aufrasten. Die Kappe trägt ein Dichtorgan 32, das hier als Dorn ausgeführt ist, der sich vorzugsweise in die Mündung der Ausgabeöffnung einspreizen oder gegen diese anpressen lässt, über die das zu applizierende Fluid in den Borstenbesatz ausgegeben wird.

Auf diese Art und Weise wird die Mündung geschützt und vorzugsweise auch abgedichtet. Das ist besonders dann praktisch, wenn die Kartusche nicht nur eine Monodose-Einheit darstellt und/oder das zu applizierende Fluid unmittelbar in die Kartusche abgefüllt wird, ohne Verwendung einer Blase.

Eine ähnliche Kappe kann auch dann zum Einsatz kommen, wenn beispielsweise eine Kartusche zum Einsatz kommt, die einen Borstenbesatz trägt, wie ihn die Fig. 2 zeigt. Der Dorn nimmt dann, zumindest an seinem äußeren Ende, vorzugsweise die Gestalt eines Rohrs an, das über die Borste gestülpt wird, über welche das zu applizierende Fluid ausgegeben wird - vorzugweise so, dass die besagte Hülsenpartie die hohle Borste dichtend umgreift.

### Bezugszeichenliste

- 1: Applikator
- 2: Applikatorhauptgehäuse
- 3: Kartusche
- 4: Borste
- 5: Borstenkanal
- 6: Kartuscheninhalt
- 7: Kartuschenkörper
- 8: Kartuschenkolben
- 9: Dorn
- 10: Borstenbasisschicht
- 11: Motor
- 12: Druckorgan
- 13: Batterie
- 14: Batteriefachdeckel
- 15: Federelement
- 16: Schalter
- 17: Gehäuseoberteil
- 18: Blase
- 19: nicht vergeben
- 20: Lampe
- 21: Riegel
- 22: Ringkragen
- 23: nicht vergeben
- 24: Elastomerspitze
- 25: Kappe
- 26: Vorsprung
- 27: Schiebeelement
- 28: nicht vergeben
- 29: nicht vergeben
- 30: Ringkragen
- 31: Dichtung vorzugsweise in Gestalt einer Schnur- oder Lippendichtung
- 32: Dichtorgan
- 33: Label (Werbeaufkleber, -aufdruck oder dergl.)
- 34: Beschriftung
- L: Applikatorlängsachse
- D: vorzugsweise angespritzte, weichelastische Dichtung
- dis: distal
- prox: proximal
- P1: Schwenkpfeil
- P2: Druckpfeil
- BL: Borstenlängsachse
- Lmax: maximale Borstenlänge
- AL: Auslenkung einer Borste
- D: Dichtung
- Dmax: maximaler Borstendurchmesser
- α: Winkel

## Patentansprüche

1. Kosmetik- oder Pharmazieapplikator (1), mit einem im Pinsel außerhalb des Borstenbesatzes bereit gehaltenen Vorrat des aufzutragenden kosmetischen oder pharmazeutischen Fluids, einem Pumpmechanismus zur Ausgabe des Fluids durch oder in den Borstenbesatz des Pinsels, wobei der Borstenbesatz des Pinsels fester Bestandteil einer einen Vorrat des Fluids bereithaltenden Kartusche (3) ist, die bei Gebrauch in einer Kammer des Applikators (1) angeordnet ist und die vorzugsweise werkzeuglos in die hierfür vorgesehene Kammer des Applikators (1) ein- und wieder ausgebaut werden kann, **dadurch gekennzeichnet, dass** die Kartusche (3) im ungebrauchten Zustand in ihrem Inneren eine Blase (18) beherbergt, die aus einer dicht verschlossenen Hülle, vorzugsweise in Gestalt einer Folienhülle oder einer Geleehülle besteht, die mit dem zu applizierenden Fluid gefüllt ist und wobei die Kartusche (3) aus einem härteren ersten Kunststoff besteht und die Borsten (4) aus einem zweiten, weicheren Kunststoff bestehen, wobei die Borsten (4) an die Kartusche (3) angespritzt und dadurch unlösbar mit der Kartusche (3) verbunden sind.

2. Applikator nach Anspruch 1, **dadurch gekennzeichnet, dass** die Kartusche eine Einwegkartusche ist.

3. Applikator nach Anspruch 1, **dadurch gekennzeichnet, dass** die Kartusche mindestens einen in ihren Innenraum vorstehenden Dorn aufweist, der die Hülle aufsticht, sobald der Pumpmechanismus zur Ausgabe des Kosmetikums in Gang gesetzt wird.

4. Applikator nach Anspruch 3, **dadurch gekennzeichnet, dass** der Dorn einen nicht-runden vorzugsweise kreuzförmigen Querschnitt aufweist.

5. Applikator nach Anspruch 1, **dadurch gekennzeichnet, dass** die Kartusche im Wesentlichen aus einem härteren ersten Kunststoff besteht, der zumindest die Umfangswände und eine Stirnwand der Kartusche ausbildet, und einem weicheren, vorzugsweise weich- oder gummielastischen zweiten Kunststoff, der die Borsten bildet, die unlösbar mit der Kartusche verbunden sind.

6. Applikator nach einem der vorhergehenden Ansprüche, **dadurch gekennzeichnet, dass** der Borstenbesatz zumindest eine und vorzugsweise nur eine Borste umfasst, die innen hohl ist und über den so gebildeten Borstenkanal den das zu applizierende Fluid beherbergenden Innenraum der Kartusche mit der Umgebung des Kosmetikpinsels verbindet.

7. Applikator nach einem der vorhergehenden Ansprüche, **dadurch gekennzeichnet, dass** der Borstenkanal so eng ausgebildet ist, dass das in der Kartusche enthaltene Fluid vorzugsweise durch die Kapillarwirkung des Borstenkanals daran gehindert wird, über den Borstenkanal nach außen auszutreten, solange der Kartuscheninhalt nicht unter Überdruck gesetzt wird.

8. Applikator nach einem der vorhergehenden Ansprüche, **dadurch gekennzeichnet, dass** die Innenseite der Stirnwand der Kartusche zumindest teilweise mit einer Schicht aus dem die Borsten bildenden Kunststoff bedeckt ist, wobei diese Schicht über örtliche Perforationen des die Kartusche bildenden Kunststoffs mit den Borsten einstofflich und einstückig in Verbindung steht.

9. Applikator nach einem der vorhergehenden Ansprüche, **dadurch gekennzeichnet, dass** die Kartusche auf der ihrem Borstenbesatz abgewandten Seite einen nach Art eines Kolbens verschiebbar geführten Boden bzw. Kartuschenkolben besitzt, wobei dieser vorzugsweise aus einem dritten Kunststoff gefertigt ist, dessen Elastizität zwischen dem die Borsten bildenden Kunststoff und dem die Umfangswand der Kartusche bildenden Kunststoff liegt.

10. Applikator nach einem der vorhergehenden Ansprüche, **dadurch gekennzeichnet, dass** die die Kartuschen aufnehmende Kammer des Pinsels zugänglich ist, indem ein Teil des Pinselkörpers um eine im Wesentlichen zur Pinsellängsachse senkrechte Scharnierachse gegenüber einem anderen Teil des Pinselkörpers aufgeklappt werden kann.

11. Applikator nach einem der vorhergehenden Ansprüche, **dadurch gekennzeichnet, dass** in den Pinselkörper ein Vibrator eingebaut ist, der den Pinsel und seinen Borstenbesatz vorzugsweise im Wesentlichen in Richtung seiner Längsachse vibrieren lässt.

12. System zum Auftragen von Kosmetikum, bestehend aus einem Kosmetik- oder Pharmazieapplikator (1) nach einem der vorhergehenden Ansprüche und einer Mehrzahl von werkzeuglos nicht nachfüllbaren Kartuschen (3), die jeweils einen Kosmetikvorrat enthalten und an die Kartuschenaufnahmekammer des Applikators angepasst sind.

13. System zum Auftragen von Kosmetikum gemäß Anspruch 12, **dadurch gekennzeichnet, dass** mehrere der Kartuschen in einer Blisterverpackung zu einem Kartuschenvorrat zusammengefasst gehalten werden, wobei die Blisterverpackung so gestaltet ist, dass jede der Kartuschen einzeln aus der Blisterverpackung entnommen werden kann, ohne andere Kartuschen bereits einem weitgehend ungehinderten Luftzutritt auszusetzen.

## Claims

1. A cosmetics or pharmaceutics applicator (1), comprising a supply of the cosmetic or pharmaceutical fluid held ready in the brush outside the bristle covering, a pump mechanism for discharging the fluid through or into the bristle covering of the brush, wherein the bristle covering of the brush is an integral component of a cartridge (3), which holds a supply of the fluid ready, is disposed in a chamber of the applicator (1) during use, and which, preferably without any tools, can be mounted in the chamber of the applicator (1) provided for this purpose and removed therefrom, **characterized in that** the cartridge (3), in the unused state, accommodates in its interior a bladder (18) consisting of a tightly sealed sheath, preferably in the form of a film sheath or jelly sheath, which is filled with the fluid to be applied, and wherein the cartridge (3) consists of a harder first plastic and the bristles (4) consist of a second softer plastic, wherein the bristles (4) are molded onto the cartridge (3) and thus non-detachably connected to the cartridge (3).

2. The applicator according to claim 1, **characterized in that** the cartridge is a disposable cartridge.

3. The applicator according to claim 1, **characterized in that** the cartridge has at least one mandrel, which protrudes into its interior and pierces the sheath as soon as the pump mechanism is set into operation for discharging the cosmetic.

4. The applicator according to claim 3, **characterized in that** the mandrel has a non-round, preferably cross-shaped, cross section.

5. The applicator according to claim 1, **characterized in that** the cartridge substantially consists of a harder first plastic which forms at least the circumferential walls and an end wall of the cartridge, and of a softer, preferably soft-elastic or rubber-elastic second plastic forming the bristles that are non-detachably connected to the cartridge.

6. The applicator according to any one of the preceding claims, **characterized in that** the bristle covering includes at least one and preferably only one bristle, which is hollow inside and, via the bristle duct formed in this way, connects the interior of the cartridge accommodating the fluid to be applied with the environment of the cosmetic brush.

7. The applicator according to any one of the preceding claims, **characterized in that** the bristle duct is configured to be so narrow that the fluid contained in the cartridge, preferably due to the capillary effect of the bristle duct, is prevented from escaping to the outside via the bristle duct as long as the cartridge contents are not subjected to overpressure.

8. The applicator according to any one of the preceding claims, **characterized in that** the inner face of the end wall of the cartridge is at least partially covered with a layer of the plastic forming the bristles, wherein this layer is in connection with the bristles by a single material and integrally via local perforations of the plastic forming the cartridge.

9. The applicator according to any one of the preceding claims, **characterized in that** the cartridge, on the side thereof facing away from its bristle covering, has a bottom or cartridge piston guided in a displaceable manner like a piston, wherein that is preferably made from a third plastic whose elasticity is between the plastic forming the bristles and the plastic forming the circumferential wall of the cartridge.

10. The applicator according to any one of the preceding claims, **characterized in that** the chamber of the brush accommodating the cartridges is accessible by it being possible that a part of the brush body can be swung open relative to another part of the brush body about a hinge axis that is substantially perpendicular to the longitudinal brush axis.

11. The applicator according to any one of the preceding claims, **characterized in that** a vibrator is incorporated into the brush body, which makes the brush and its bristle covering vibrate, preferably substantially in the direction of its longitudinal axis.

12. A system for applying a cosmetic, consisting of a cosmetics or pharmaceutics applicator (1) according to any one of the preceding claims and a plurality of cartridges (3), which cannot be refilled without tools, which respectively contain a cosmetics supply and which are adapted to the cartridge accommodating chamber of the applicator.

13. A system for applying a cosmetic according to claim 12, **characterized in that** several of the cartridges, combined to form a cartridge supply, are held in a blister pack, wherein the blister pack is configured such that each of the cartridges can be individually removed from the blister pack without already exposing other cartridges to a largely unimpeded admission of air.

## Revendications

1. Applicateur cosmétique ou pharmaceutique (1) doté d'une réserve, déjà contenue dans le pinceau à l'extérieur de la garniture de poils, du fluide cosmétique ou pharmaceutique à appliquer, d'un mécanisme de pompe pour distribuer le fluide à travers ou dans la garniture de poils du pinceau, dans lequel la garniture de poils du pinceau fait intégralement partie d'une cartouche (3) qui fournit la réserve du fluide, qui, en utilisation, est disposée dans une chambre de l'applicateur (1) et qui peut être, de préférence sans outil, montée dans et démontée hors de la chambre de l'applicateur (1) prévue à cet effet, **caractérisé en ce que** la cartouche (3), dans l'état non utilisé, renferme à l'intérieur une poche (18) qui se compose d'une enveloppe fermée de manière étanche, de préférence sous la configuration d'une enveloppe de film ou d'une enveloppe de gelée, qui est remplie avec le fluide à appliquer, dans lequel la cartouche (3) est composée d'une première matière synthétique plus dure et les poils (4) sont constitués en une deuxième matière synthétique plus souple, les poils (4) étant moulés par injection sur la cartouche (3) et donc reliés à la cartouche (3) de manière indétachable.

2. Applicateur selon la revendication 1, **caractérisé en ce que** la cartouche est une cartouche à usage unique.

3. Applicateur selon la revendication 1, **caractérisé en ce que** la cartouche présente au moins une pointe se projetant dans son volume intérieur, qui perce l'enveloppe dès que le mécanisme de pompe est activé pour distribuer le produit cosmétique.

4. Applicateur selon la revendication 3, **caractérisé en ce que** la pointe présente une section qui n'est pas ronde et qui est de préférence cruciforme.

5. Applicateur selon la revendication 1, **caractérisé en ce que** la cartouche se compose sensiblement d'une première matière synthétique plus dure qui forme au moins les parois périphériques et une paroi frontale de la cartouche, et d'une deuxième matière synthétique plus souple ou présentant l'élasticité du caoutchouc qui forme les poils qui sont reliés à la cartouche de manière indétachable.

6. Applicateur selon l'une des revendications précédentes, **caractérisé en ce que** la garniture de poils comprend au moins un et de préférence un seul poil qui est creux à l'intérieur et qui relie, via le canal de poil ainsi réalisé, le volume intérieur de la cartouche abritant le fluide qu'il s'agit d'appliquer à l'environnement du pinceau cosmétique.

7. Applicateur selon l'une des revendications précédentes, **caractérisé en ce que** le canal de poil est réalisé de manière si étroite que le fluide contenu dans la cartouche est empêché de s'écouler vers l'extérieur via le canal de poil, de préférence par effet capillaire du canal de poil, tant que le contenu de la cartouche n'est pas mis sous surpression.

8. Applicateur selon l'une des revendications précédentes, **caractérisé en ce que** la face intérieure de la paroi frontale de la cartouche est recouverte au moins en partie d'une couche réalisée dans la matière synthétique formant les poils, dans lequel cette couche est en liaison de matière et d'une seul tenant avec les poils via des perforations locales de la matière synthétique constituant la cartouche.

9. Applicateur selon l'une des revendications précédentes, **caractérisé en ce que**, sur sa face tournée vers sa garniture de poils, la cartouche possède un fond guidé de manière déplaçable à la manière d'un piston ou un piston de cartouche, dans lequel celui-ci est fabriqué de préférence dans une troisième matière synthétique dont l'élasticité se trouve entre celle de la matière synthétique constituant les poils et celle de la matière synthétique constituant la paroi périphérique de la cartouche.

10. Applicateur selon l'une des revendications précédentes, **caractérisé en ce que** la chambre du pinceau qui reçoit les cartouches est accessible en ouvrant par basculement une partie du corps du pinceau autour d'un axe de charnière sensiblement perpendiculaire à l'axe longitudinal du pinceau par rapport à une autre partie du corps de pinceau.

11. Applicateur selon l'une des revendications précédentes, **caractérisé en ce qu'**un vibreur est intégré dans le corps de pinceau, qui fait vibrer le pinceau et sa garniture de poils de préférence sensiblement en direction de son axe longitudinal.

12. Système d'application d'un produit cosmétique, composé d'un applicateur cosmétique ou pharmaceutique (1) selon l'une des revendications précédentes et d'une pluralité de cartouches (3) non reremplissables, utilisables sans outil, qui contiennent chacune une réserve de produit cosmétique et qui sont adaptées à la chambre de réception de cartouches de l'applicateur.

13. Système d'application d'un produit cosmétique selon la revendication 12, **caractérisé en ce que** plusieurs des cartouches sont regroupées dans un conditionnement blister en une réserve de cartouches, dans lequel le conditionnement blister est conçu de sorte que chacune des cartouches puisse être retirée individuellement du conditionnement blister sans exposer déjà d'autres cartouches à une arrivée d'air largement sans entrave.
